# EUROPEAN PATENT APPLICATION

(11) **EP 4 265 228 A1**
(43) Date of publication of application: **25.10.2023**
(21) Application number: 21905287.5
(22) Date of filing: 21.10.2021
(51) Int. Cl.: A61F 2/90

(54) **COVERED STENT**

(30) Priority: 18.12.2020 CN 202011507209
(71) Applicant: Lifetech Scientific (Shenzhen) Co., Ltd., Shenzhen, Guangdong 518063 (CN)
(72) Inventor: TANG, Jiangfeng, Shenzhen, Guangdong 518063 (CN); QIN, Cuifang, Shenzhen, Guangdong 518063 (CN)
(74) Representative: Prinz & Partner mbB
(86) International application number: PCT/CN2021/125235
(87) International publication number: WO 2022/127358

(57) **Abstract**

A covered stent (10), including a membrane (12) and a support (11, 21, 31, 41, 51), where the membrane (12) includes a first membrane (121, 221, 321, 421, 521) and a second membrane (122, 222, 322, 422, 522); the support (11, 21, 31, 41, 51) is arranged between the first membrane (121, 221, 321, 421, 521) and the second membrane (122, 222, 322, 422, 522); the first membrane (121, 221, 321, 421, 521) covers an end portion of the support (11, 21, 31, 41, 51); the membrane (12) further includes a third membrane (123, 223, 323, 423, 523); the third membrane (123, 223, 323, 423, 523) is arranged on an outer surface of the support (11, 21, 31, 41, 51) and is close to an end portion of the covered stent (10); and a cross section where the third membrane (123, 223, 323, 423, 523) is located includes two layers of membranes arranged on the outer surface of the support (11, 21, 31, 41, 51) and one layer of membrane arranged on an inner surface of the support (11, 21, 31, 41, 51). The covered stent (10) locally includes the first membrane (121, 221, 321, 421, 521), the second membrane (122, 222, 322, 422, 522) and the third membrane (123, 223, 323, 423, 523); the first membrane (121, 221, 321, 421, 521) covers the proximal end of the support (11, 21, 31, 41, 51), and the cross section where the third membrane (123, 223, 323, 423, 523) is located includes three layers of membranes, two of which are arranged on the outer surface of the support (11, 21, 31, 41, 51), which reduces the difficulty of sheathing the covered stent (10) without affecting an inner diameter of the covered stent (10), and also ensures that the covered stent (10) has a good anchoring performance and sealing performance.

## Description

### Technical Field

The present disclosure relates to the technical field of medical instruments, specifically to a covered stent.

### Background

Minimally invasive surgery for vascular repair with a covered stent is widely used to treat aortic vascular diseases due to its minimal trauma, rapid recovery and good instant effect. At present, a covered stent with polytetrafluoroethylene (PTFE) membranes is one of the mainstream covered stents in the market. The PTFE membrane is combined with a metal skeleton by heat fusion. Generally, the metal skeleton is between and covered by an inner layer of PTFE membrane and an outer layer of PTFE membrane. After the covered stent is implanted into a blood vessel, the proximal end of the stent will continue to be flushed by blood. Since the covered stent with the PTFE membranes has inner and outer layers of membrane structures which are combined by heat fusion, in order to prevent an edge of the membrane on the proximal end from being flushed away by the blood, resulting in separation of the inner and outer membranes, an edge of the proximal end of the covered stent is usually flanged. During manufacturing, the inner layer of membrane is flanged outwardly on a mold to cover the proximal end of the metal skeleton. After the coverage of the outer layer of membrane is completed, the membrane is subjected to thermal treatment to fuse the inner layer of membrane and the outer layer of membrane. A semi-finished product is removed from the mold. The outer layer of membrane is then turned inwards, and the edge is ironed by electric soldering iron, so that an outer layer of flanging is bonded with the inner layer of membrane. In this way, it is generally difficult to firmly bond the outer layer of flanging to the inner layer of membrane. At the same time, there are four layers of membranes at a proximal end portion of the covered stent, so the proximal end portion is thicker than other portions. Furthermore, the inner diameter of the proximal end of the stent is reduced. In addition, in order to improve the sealing performance of the proximal end of the stent, denser smaller waveform rings will usually be braided. Therefore, a metal waveform ring of the proximal end of the covered stent has a larger wave number, and the membrane is thicker. Furthermore, a developing point is added at the edge of the membrane of the proximal end, so that the volume after compression is larger than that of other positions. Under the same conditions, it is difficult for sheathing, or a sheath expands after being placed and is difficult to release.

### Summary

Based on this, it is necessary to design a covered stent which is easy to sheath and will not reduce the anchoring performance and the sealing performance.

The present disclosure provides a covered stent, including a membrane and a support, wherein the membrane includes a first membrane and a second membrane; the support is arranged between the first membrane and the second membrane; the first membrane covers an end portion of the support; the membrane further includes a third membrane; the third membrane is arranged on an outer surface of the support and is close to an end portion of the covered stent; and a cross section where the third membrane is located comprises two layers of membranes arranged on the outer surface of the support and one layer of membrane arranged on an inner surface of the support.

In one embodiment, an end portion of the first membrane bypasses the end portion of the support and is then folded towards the second membrane; and the third membrane includes a folded portion of the first membrane.

In one embodiment, a proximal end of the second membrane is flush with a proximal end of the support, or the proximal end of the second membrane is closer to a distal end of the covered stent than the proximal end of the support.

In one embodiment, the first membrane bypasses the proximal end of the support and is then folded towards the distal end; the folded portion of the first membrane forms the second membrane; and the third membrane is arranged on an outer surface of the second membrane, or the third membrane is arranged between an inner surface of the second membrane and the outer surface of the support.

In one embodiment, the covered stent includes a plurality of sections of third membranes arranged at intervals in an axial direction.

In one embodiment, the covered stent includes a plurality of sections of third membranes, and two adjacent sections of the third membranes are stacked end to end.

In one embodiment, among the two adjacent sections of third membranes, a distal end portion of the third membrane close to the proximal end covers a proximal end portion of the third membrane close to the distal end.

In one embodiment, the support includes a sealing member located at the proximal end portion of the support; and a distal end of the sealing member is closer to the proximal end of the covered stent than a proximal end of the third membrane.

In one embodiment, the distance between a distal end of the third membrane and the proximal end of the support is greater than or equal to 15 mm.

The covered stent provided by the present disclosure locally includes the first membrane, the second membrane and the third membrane; the first membrane covers the proximal end of the support, and the cross section where the third membrane is located includes three layers of membranes, two of which are arranged on the outer surface of the support, which reduces the difficulty of sheathing the covered stent without affecting an inner diameter of the covered stent, and also ensures that the covered stent has a good anchoring performance and sealing performance.

### Brief Description of the Drawings

Fig. 1 is a schematic structural diagram of a covered stent according to one embodiment of the present disclosure;
Fig. 2 is a schematic diagram of a membrane layout of a covered stent according to one embodiment of the present disclosure;
Fig. 3 is a schematic diagram of a membrane layout of a covered stent according to another embodiment of the present disclosure;
Fig. 4 is a schematic diagram of a membrane layout of a covered stent according to still another embodiment of the present disclosure;
Fig. 5 is a schematic diagram of a membrane layout of a covered stent according to yet another embodiment of the present disclosure; and
Fig. 6 is a schematic diagram of a membrane layout of a covered stent according to still another embodiment of the present disclosure.

### Detailed Description of the Disclosure

In order to better understand the technical solutions and beneficial effects of the present disclosure, the solutions of the present disclosure are further described in detail in combination with specific embodiments. The following specific embodiments are only part of the embodiments of the present disclosure, and this specification cannot enumerate all possible embodiments.

Unless otherwise specified, the terms of the present disclosure are commonly used by those skilled in the art.

In the field of medical devices, it is generally defined that a direction of blood inflow is a "proximal end" and a direction of blood outflow is a "distal end". That is, for a covered stent of the present disclosure, during use, the blood flows in from the proximal end of the covered stent and flows out from the distal end of the covered stent. The proximal end and distal end of a certain component and a relative positional relationship between two components are described according to this.

### Embodiment I

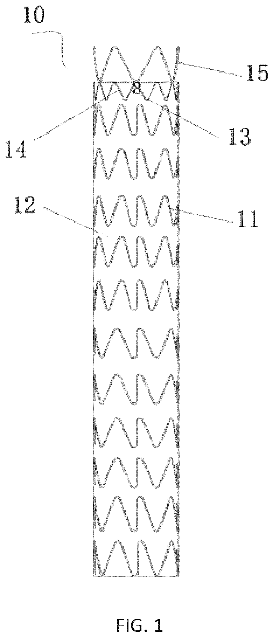

As shown in Fig. 1, a covered stent 10 of the present disclosure is of an overall hollow tubular structure, both ends of which are respectively provided with openings. The covered stent 10 includes a membrane 12 and a support 11. The support 11 includes a plurality of axially arranged waveform rings which are connected by the membrane 12. The support 11 also includes a sealing member 14 arranged at the proximal end. Compared with waveform rings of other portions, the sealing member 14 has braiding wires with a smaller diameter, a smaller height and a larger braiding density, so that the sealing member arranged at a proximal end portion can play a better role of sealing the end portion.

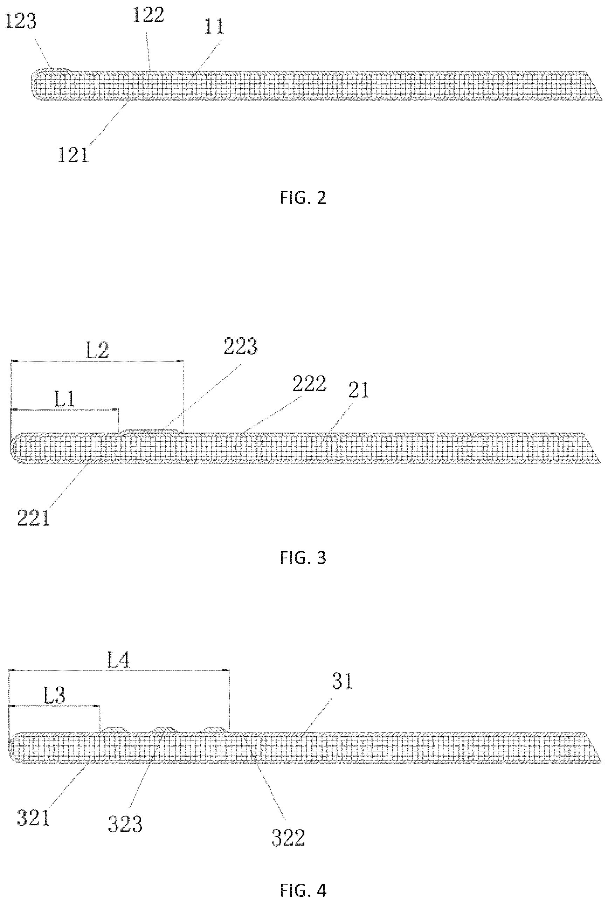

Referring to Fig. 1 and Fig. 2, the membrane includes a first membrane 121 and a second membrane 122. The support 11 is arranged between the first membrane 121 and the second membrane 122. In this embodiment, the first membrane 121 is arranged on an inner surface of the covered stent 10, and the first membrane 121 covers an end portion of the support 11. In this embodiment, the first membrane 121 covers the proximal end of the sealing member 14. The second membrane 122 is arranged on an outer surface of the covered stent 10.

It can be understood that the first membrane can cover only the proximal end of the support, or cover both the proximal end and the distal end of the support. The distal end of the membrane will not directly be forward flushed by the blood and is generally not provided with a sealing member, a developing point is also relatively small, and the assembly difficulty is relatively low, so that the present disclosure does not make more restrictions on the layout of the membrane at the distal end of the covered stent.

In order to enhance the anchoring performance of the covered stent 10, the covered stent 10 also includes an anchoring member 15 arranged at the proximal end of the membrane 12. The anchoring member 15 is a bare waveform ring, which directly contacts with a vascular tissue after implantation. In order to further improve the anchoring performance, barbs can also be arranged on the anchoring member 15.

The covered stent 10 also includes an imaging piece 13. The imaging piece 13 includes a material with a good imaging characteristic, which can be arranged at the proximal end, distal end and other specific positions to allow imaging of the covered stent.

It can be understood that in other embodiments, the covered stent may not be provided with a sealing and an anchoring member.

In order to facilitate an understanding of the layout characteristic of the membrane on the covered stent of the present disclosure, Fig. 2 to Fig. 6 only schematically show positional relationships between the membrane and the support, and the support is only represented by the simplest structure.

As shown in Fig. 2, the covered stent 10 of this embodiment includes a first membrane 121, a second membrane 122 and a third membrane 123. The proximal end of the second membrane 122 is flush with the proximal end of the support 11; the proximal end portion of the first membrane 121 bypasses the proximal end of the support 11 and covers the proximal end portion of the second membrane 122 to cover the proximal end of the support 11. A portion of the first membrane 121 that bypasses the proximal end portion of the support 11 and is then folded towards the second membrane 122 is the third membrane 123. That is, the third membrane 123 and the support 11 sandwich the proximal end portion of the second membrane 122. In this manner, the cross section where the third membrane 123 is located includes three layers of membranes, and two layers of membranes are located on the outer surface of the support 11, and one layer of membrane is located on the inner surface of the support 11. However, the cross section of the covered stent 10 far away from the third membrane 123 only includes at most two layers of membranes. In other words, on any cross section where the third membrane is located, there are only two layers of membranes arranged on the outer surface of the support 11. Preferably, on any cross section of the covered stent, no more than three layers of membranes are arranged on the inner and outer surfaces of the support. In this manner, the dimension of the proximal end of the covered stent is reduced, and the overall thickness of the covered stent is also reduced. In addition, it should be understood that in the field of medical devices, when the covered stent is prepared, all portions of the same covered stent have a uniform thickness.

In order to ensure the anchoring performance and sealing performance of the proximal end of the covered stent 10, it is preferred that the distance between the distal end of the third membrane 123 and the proximal end of the support 11 is greater than or equal to 15 mm.

When the covered stent 10 of this embodiment is manufactured, the first membrane 121 first surrounds a mold rod, and the support 11 is then placed. Furthermore, a part with a certain length of the proximal end of the first membrane 121 is not covered by the support 11. The second membrane 122 then surrounds the support 11, so that the proximal end of the second membrane 12 is just flush with the proximal end of the support 11, or is slightly beyond the proximal end of the support 11 (considering that a membrane material will be slightly shortened during thermal treatment). The portion of the proximal end of the first membrane 121 that is not covered by the support 11 is folded outwardly to cover both the proximal end of the support and the proximal end portion of the second membrane 122. Finally, the mold rod, the membrane and the support are placed into a thermal treatment equipment together for thermal treatment, so that the first membrane 121 and the second membrane 122 are overall fused to fix the support and also form the third membrane 123.

In this embodiment, by means of simply folding the first membrane outward, the proximal end of the membrane has at most three layers of membranes. Two layers of membranes are located on an outer side of the support, and one layer of membrane is located on an inner side of the support. Furthermore, the second membrane is not folded inward, so that the inner diameter of the proximal end of the covered stent is not affected, and there is no risk that the membrane is flushed away. In addition, in the covered stent of the present disclosure, the membrane can be fixed by one thermal treatment. The membrane stability is better, and the machining steps are also reduced.

### Embodiment II

The covered stent of this embodiment is substantially the same as that of Embodiment I, and a difference lies in the layout of the membrane. As shown in Fig. 3, the covered stent of this embodiment includes a first membrane 221, a second membrane 222 and a third membrane 223. The first membrane 221 is arranged on an inner surface of a support 21; a second membrane 222 is arranged on an outer surface of the support 21; and the proximal end of the second membrane 222 is closer to the distal end of the covered stent than a proximal end of the support 21. The proximal end portion of the first membrane 221 bypasses the proximal end of the support 21 and is folded towards the second membrane 222 to cover both a proximal end portion of the second membrane 222 and the proximal end of the support 21. A portion of the first membrane 221 folded to cover the second membrane 222 is the third membrane 223.

In this embodiment, the distance between the proximal end of the third membrane 223 and the proximal end of the support 21 is L1, and the distance between the distal end of the third membrane 223 and the proximal end of the support 21 is L2. In order to give consideration to the anchoring performance and sealing performance of the proximal end of the covered stent, and to minimize the difficulty in assembling the covered stent, in this embodiment, L1 is preferably greater than an axial length of the sealing member, that is, the distal end of the sealing member is closer to the proximal end of the covered stent than the proximal end of the third membrane 223, so that the third membrane 223 does not cover the sealing member, which reduces the overall dimension of the position where the sealing member is located. In addition, L2 is preferably greater than or equal to 15 mm. For example, the axial length of the sealing member is about 7-8 mm, so the axial length of the third membrane can be 7-8 mm.

Manufacturing the covered stent of this embodiment is similar to the manufacturing of the covered stent of Embodiment I, except that during covering, the proximal end of the second membrane shall not cover the sealing member, and the proximal end of the second membrane is slightly close to the distal end of the sealing member. The subsequent covering process is the same as that in Embodiment I, and will not be repeated here.

In this embodiment, since L1 is greater than the axial length of the sealing member, that is, since the third membrane is staggered from the sealing member, that is, since the third membrane and the sealing member are not on the same cross section, the position where the sealing member is located only includes two layers of membranes, which further reduces the dimension of the proximal end of the covered stent and reduces the difficulty of loading.

### Embodiment III

The overall structure of the covered stent of this embodiment is substantially the same as that of the covered stent of Embodiment I, and a difference lies in the layout of the membrane. As shown in Fig. 4, the covered stent of this embodiment includes a first membrane 321, a second membrane 322 and a third membrane 323. The first membrane 321 is arranged on an inner surface of a support 31, and the second membrane 322 is arranged on an outer surface of the support 31. The first membrane 321 bypasses the proximal end of the support 31 and is folded towards the distal end to form the second membrane 322. That is, the first membrane 321 and the second membrane 322 are of an integrated structure, which is an overall membrane. The third membrane 323 is arranged on an outer surface of the second membrane 322, and is close to the proximal end of the support 31. In this embodiment, the covered stent includes a plurality of sections of third membranes 323, and the plurality of sections of third membranes 323 are axially arranged at intervals around a periphery of the covered stent. Distances between the multiple sections of third membranes can be the same or different. Preferably, the distances between multiple sections of third membranes gradually increase from the proximal end to the distal end.

In this embodiment, the distance between the proximal end of the third membrane 323 at the most proximal end and the proximal end of the support 31 is L3, and the distance between the distal end of the third membrane 323 at the most distal end and the proximal end of the support 31 is L4. Preferred ranges of L3 and L4 in this embodiment can refer to the preferred ranges of L1 and L2 in Embodiment II respectively, and will not be repeated here.

When the covered stent of this embodiment is manufactured, the first membrane 321 first surrounds a mold rod, and the support 31 is then placed. Furthermore, a part with a certain length of the proximal end of the first membrane 321 is not covered by the support 31, and the length of the reserved portion is slightly greater than an axial length of the support. The third membrane then surrounds the proximal end portion of the outer surface of the outward folded first membrane (i.e. the second membrane). The distance between the proximal end of the third membrane and the proximal end of the support is controlled at L3, so that more sections of third membranes can be surrounded, but it is required that the distance L4 between the distal end of the third membrane located at the most distal end and the proximal end of the support meets the above requirement. Finally, the mold rod, the membrane and the support are placed into thermal treatment equipment together for thermal treatment, so that the membranes are overall fused and connected to fix the support.

It can be understood that in other embodiments, only one section of third membrane can also be provided.

In this embodiment, the sealing performance between the membrane at the proximal end of the covered stent and a vascular wall can be further improved by means of arranging the multiple sections of third membranes, and the roughness of the outer surface of the covered stent can also be increased, the friction between the outer surface of the covered stent and a blood vessel can be increased, and an anchoring force at the proximal end of the covered stent can be improved.

### Embodiment IV

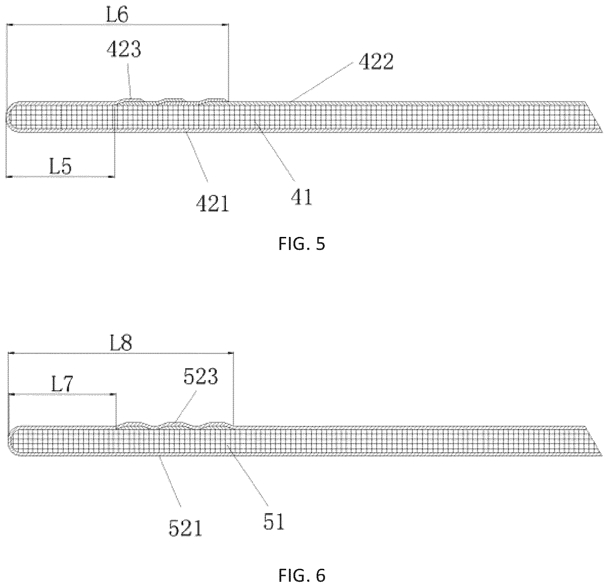

The overall structure of the covered stent of this embodiment is substantially the same as that of the covered stent of Embodiment I, and a difference lies in the layout of the membrane. As shown in Fig. 5, the covered stent of this embodiment includes a first membrane 421, a second membrane 422 and a third membrane 423. The first membrane 421 is arranged on an inner surface of a support 41; a second membrane 422 is arranged on an outer surface of the support 41; and the proximal end of the second membrane 422 is closer to a distal end of the covered stent than the proximal end of the support 41.

The covered stent of this embodiment includes a plurality of sections of third membranes 423, and two adjacent sections of the third membranes 423 are stacked end to end. The proximal end portion of the first membrane 421 bypasses the proximal end of the support 41 and is folded towards the distal end to form the third membrane 423 located at the most proximal end. Other third membranes 423 all include covered portions and uncovered portions, for the convenience of description, which are referred to as "first portion" (or proximal end portion) and "second portion" (or distal end portion). Among the multiple sections of third membranes 423, the second portion of the third membrane 423 located at the most distal end covers the proximal end portion of the second membrane 422. The first portion of the third membrane 423 in the middle is covered by the second portion of the adjacent third membrane 423 at the proximal end. The second portion of the third membrane 423 in the middle covers the first portion of the adjacent third membrane 423 at the distal end.

It can be understood that in other embodiments, in two adjacent third membranes, the second portion of the third membrane close to the proximal end is covered by the first portion of the third membrane close to the distal end. In this way, the edge of the proximal end of the third membrane is not covered. Therefore, in the early stage of the implantation of the covered stent, the blood is in contact with the edge of the proximal end of the third membrane to speed up endothelialization and improve the anchoring performance of the proximal end of the covered stent.

In this embodiment, the distance between the proximal end of the third membrane 423 at the most proximal end and the proximal end of the support 41 is L5, and the distance between the distal end of the third membrane 423 at the most distal end and the proximal end of the support 41 is L6. Preferred ranges of L5 and L6 in this embodiment can refer to the preferred ranges of L1 and L2 in Embodiment II respectively, and will not be repeated here.

When the covered stent of this embodiment is manufactured, the first membrane 421 first surrounds a mold rod, and the support 41 is then placed. Furthermore, a part with a certain length of the proximal end of the first membrane 421 is not covered by the support 41. The second membrane 422 then surrounds the support 41, and the proximal end of the second membrane 422 is far away from the proximal end of the support. The proximal end portion of the first membrane 421 is folded outward, ensuring that there is still a part of the support not covered between the proximal end of the first membrane 421 and the proximal end of the second membrane. One section of third membrane 423 surrounds the support 41, so that the distal end portion of the third membrane 423 covers the proximal end portion of the second membrane 422, and the proximal end portion of the third membrane 423 is covered by the distal end portion of the first membrane 421. Alternatively, a plurality of sections of third membranes 423 surround the support, but it is required that in two adjacent third membranes 423, the distal end portion of the third membrane 423 close to the proximal end covers the proximal end portion of the third membrane 423 close to the distal end. Finally, the mold rod, the membrane and the support are placed into thermal treatment equipment together for thermal treatment, so that the membranes are overall fused and connected to fix the support. Furthermore, a portion of the folded proximal end portion of the first membrane 421 covering the proximal end of the adj acent third membrane also forms the third membrane.

The covered stent of this embodiment includes multiple sections of third membranes stacked end to end, so that the sealing performance of the proximal end of the covered stent can be further improved. Meanwhile, the roughness of the outer surface of the covered stent can also be increased, the friction between the outer surface of the covered stent and a blood vessel can be increased, and an anchoring force at the proximal end of the covered stent can be improved. In addition, the proximal ends of the third membranes are covered, it is more convenient for loading, and there is no risk that an edge of a membrane is upwarped.

### Embodiment V

The overall structure of the covered stent of this embodiment is substantially the same as that of the covered stent of Embodiment I, and a difference lies in the layout of the membrane. As shown in Fig. 6, the covered stent of this embodiment includes a first membrane 521, a second membrane 522 and a third membrane 523. The first membrane 521 is arranged on an inner surface of a support 51, and the second membrane 522 is arranged on an outer surface of the support 51. The first membrane 521 bypasses the proximal end of the support 51 and is folded towards the distal end to form the second membrane 522. That is, the first membrane 521 and the second membrane 522 are of an integrated structure, which is an overall membrane. The third membrane 523 is arranged on an inner surface of the second membrane 522, and is close to the proximal end of the support 51. In this embodiment, the covered stent includes a plurality of sections of third membranes 523, and the plurality of sections of third membranes 523 are axially arranged at intervals around a periphery of the covered stent.

In this embodiment, the distance between the proximal end of the third membrane 523 at the most proximal end and the proximal end of the support 51 is L7, and the distance between the distal end of the third membrane 523 at the most distal end and the proximal end of the support 51 is L8. Preferred ranges of L7 and L8 in this embodiment can refer to the preferred ranges of L1 and L2 in Embodiment II respectively, and will not be repeated here.

It can be understood that in other embodiments, only one section of third membrane can also be provided.

The method of manufacturing the covered stent of this embodiment is similar to the method of manufacturing the covered stent of Embodiment III, but a difference is that after the first membrane and the support surrounds the mold rod, the third membrane surrounds the outer side of a portion which close to the proximal end of the support; and the first membrane is then folded. Other operation steps will be omitted.

In this embodiment, the sealing performance between the membrane at the proximal end of the covered stent and a vascular wall can be further improved by means of arranging the multiple sections of third membranes, and the roughness of the outer surface of the covered stent can also be increased, the friction between the outer surface of the covered stent and a blood vessel can be increased, and an anchoring force at the proximal end of the covered stent can be improved. In addition, the third membrane is arranged between the second membrane and the support, so that the outer surface of the second membrane is naturally transitioned, which can avoid the risk of an edge of the membrane is scraped off during assembling and releasing of the covered stent.

It should be understood that the above specific embodiments are only preferred embodiments of the present disclosure, not intended to limit the present disclosure. Those skilled in the art can simply replace and combine some structures after understanding the concept of the present disclosure, and the replacements and combinations still fall within the protection scope of the present disclosure.

## Claims

1. A covered stent, comprising a membrane and a support, wherein the membrane comprises a first membrane and a second membrane; the support is arranged between the first membrane and the second membrane; the first membrane covers an end portion of the support; the membrane further comprises a third membrane; the third membrane is arranged on an outer surface of the support and is close to an end portion of the covered stent; and a cross section where the third membrane is located comprises two layers of membranes arranged on the outer surface of the support and one layer of membrane arranged on an inner surface of the support.

2. The covered stent according to claim 1, wherein an end portion of the first membrane bypasses the end portion of the support and is then folded towards the second membrane; and the third membrane comprises a folded portion of the first membrane.

3. The covered stent according to claim 2, wherein a proximal end of the second membrane is flush with a proximal end of the support, or the proximal end of the second membrane is closer to a distal end of the covered stent than the proximal end of the support.

4. The covered stent according to claim 1, wherein the first membrane bypasses the proximal end of the support and is then folded towards the distal end; the folded portion of the first membrane forms the second membrane; and the third membrane is arranged on an outer surface of the second membrane, or the third membrane is arranged between an inner surface of the second membrane and the outer surface of the support.

5. The covered stent according to claim 4, wherein the covered stent comprises a plurality of sections of third membranes arranged at intervals in an axial direction.

6. The covered stent according to claim 1, wherein the covered stent comprises a plurality of sections of third membranes, and two adjacent sections of the third membranes are stacked end to end.

7. The covered stent according to claim 6, wherein among the two adjacent sections of third membranes, a distal end portion of the third membrane close to the proximal end covers a proximal end portion of the third membrane close to the distal end.

8. The covered stent according to any one of claims 1 to 7, wherein the support comprises a sealing member located at the proximal end portion of the support; and a distal end of the sealing member is closer to the proximal end of the covered stent than a proximal end of the third membrane.

9. The covered stent according to any one of claims 1 to 7, wherein the distance between a distal end of the third membrane and the proximal end of the support is greater than or equal to 15 mm.
